# EUROPÄISCHE PATENTANMELDUNG

(11) **EP 4 796 144 A1**
(43) Veröffentlichungstag der Anmeldung: **26.08.2026**
(21) Anmeldenummer: 25224804.2
(22) Anmeldetag: 18.12.2025
(51) Int. Cl.: A61M 5/155, A61M 5/20, A61M 5/31

(54) **INJEKTIONSSYSTEM ZUR ANWENDUNG BEI OPHTHALMOLOGISCHEN EINGRIFFEN**

(30) Priorität: 21.02.2025 DE 102025106702
(71) Anmelder: Geuder AG, 69126 Heidelberg (DE)
(72) Erfinder: Müller-Albinus, Julius, 69488 Birkenau (DE)
(74) Vertreter: Ullrich & Naumann PartG mbB

(57) **Zusammenfassung**

Ein Injektionssystem zur Anwendung bei ophthalmologischen Eingriffen, insbesondere zum Injizieren eines Fluidums in das menschliche oder tierische Auge, vorzugsweise in den hinteren Augenabschnitt, umfassend einen Spritzenkörper (1) mit einem darin ausgebildeten zylindrischen Hohlraum (2) für das Fluidum, einer konus- oder zylinderförmigen Düse (3) zum Anbringen eines Schlauchs oder einer Nadel (4) an den Spritzenkörper (1) und einem am gegenüberliegenden Ende des Spritzenköpers (1) angeordneten Anschluss mit Durchgang (11) für ein Druckmedium, insbesondere Druckluft, zum düsenseitigen Ausbringen des Fluidums, wobei der Anschluss an ein offenes Ende des Spritzenköpers (1) angeflanscht bzw. anflanschbar ist.

## Beschreibung

Die Erfindung betrifft ein Injektionssystem zur Anwendung bei ophthalmologischen Eingriffen, insbesondere zum Injizieren eines Fluidums in das menschliche oder tierische Auge, vorzugsweise in den hinteren Augenabschnitt.

Injektionssysteme der hier in Rede stehenden Art umfassen einen Spritzenkörper mit einem darin ausgebildeten zylindrischen Hohlraum für ein Fluidum. An den eigentlichen Spritzenkörper schließt sich eine konus- oder zylinderförmige Düse zum Anbringen eines Schlauchs oder einer Nadel an. Am gegenüberliegenden Ende des Spritzenkörpers ist üblicherweise ein Betätigungsorgan vorgesehen, welches zum Verschieben eines Kolbens in dem zylindrischen Hohlraum dient. Durch Betätigen lässt sich das Fluidum aus dem zylindrischen Hohlraum durch die Nudel hindurch ausbringen, d.h. in das Auge injizieren.

Injektionssysteme zur Anwendung bei ophthalmologischen Eingriffen sind hinlänglich aus der Praxis bekannt. Sie werden ganz überwiegend in der Hinterabschnittschirurgie eingesetzt, d.h. bei chirurgischen Eingriffen im hinteren Augenabschnitt. Dort ist der größte Teil des Auges von dem sogenannten Glaskörper ausgefüllt. Der Glaskörper ist eine gallertartige Substanz, die aus Hyaluronsäure, Kollagen und Wasser besteht. Er füllt das Augeninnere und verleiht ihm Stabilität. Die Macula lutea, auch gelber Fleck genannt, ist die Stelle des schärfsten Sehens. Die Netzhaut (Retina) bildet die eintreffenden Lichtbündel als Bilder ab und leitet die Informationen durch den Sehnerv an das Gehirn weiter. Dort werden die Informationen verarbeitet.

Für Erkrankungen des Auges am hinteren Abschnitt werden mannigfaltige Behandlungen angeboten.

Im Falle einer Makuladegeneration werden u.a. Medikamente in bzw. an den geschädigten Bereich injiziert.

Zur operativen Behandlung von Netzhauterkrankungen wird im Rahmen der sogenannten Vitrektomie der Glaskörper entfernt und die Netzhaut wird entsprechend der Grunderkrankung z.B. mit einem Laser behandelt.

Bei der Vitrektomie wird der Glaskörper über feine Schnitte in der Hornhaut abgesaugt und durch eine Flüssigkeit oder ein Gas, das ähnliche optische Eigenschaften besitzt, ersetzt. Jede Glaskörperentfernung benötigt einen Ersatz. Häufig genügt der Austausch gegen eine Flüssigkeit, die ähnlich dem natürlichen Glaskörper ist. In einigen Fällen, z.B. bei Netzhautablösung, Makulaloch, etc., ist eine Tamponade erforderlich, um die Netzhaut an die richtigen Stellen anzudrücken. Dazu werden Gase oder Silikonöl verwendet, je nach klinischem Befund. Zum Einbringen des Silikonöls werden Injektionssysteme verwendet, wobei es derzeit üblich ist, die dazu vorgesehene Spritze über deren Kolben manuell zu bedienen. Bei relativ dickflüssigen Silikonölen oder auch schweren Ölen für die Behandlung von Löchern ist das händische Ausbringen des Öls, nämlich das Injizieren in den hinteren Augenabschnitt, mühsam. Außerdem ist es äußerst schwierig, die Ausbringrate per Hand gleichmäßig zu dosieren.

Aus der Praxis ist es auch bereits bekannt, Injektionssysteme der hier in Rede stehenden Art mit Druckluft zu betreiben, wobei dazu besondere Spritzenkörper verwendet werden. Dies ist aufwändig und schließt die Verwendung herkömmlicher Spritzen/Spritzenkörper aus.

Der Erfindung liegt die Aufgabe zugrunde, ein Injektionssystem zur Anwendung bei ophthalmologischen Eingriffen derart auszugestalten und weiterzubilden, dass ein vorgebbares, gleichmäßiges Injizieren eines Fluidums in das menschliche oder tierische Auge möglich ist. Dazu sollen die wesentlichen Bestandteile einer herkömmlichen Spritze verwendet werden, insbesondere der Spritzenkörper mit seiner spezifischen Ausgestaltung.

Voranstehende Aufgabe ist durch die Merkmale des Anspruchs 1 gelöst, wonach das Injektionssystem den Spritzenkörper einer herkömmlichen Spritze umfasst. In dem Spritzenkörper ist ein zylindrischer Hohlraum für das Fluidum ausgebildet.

Am freien Ende des Spritzenkörpers ist eine konus- oder zylinderförmige Düse vorgesehen, die zum Anbringen eines Schlauchs oder einer Nadel an den Spritzenkörper dient. Mittels Nadel kann der hinterer Augenabschnitt mühelos erreicht werden.

Am gegenüberliegenden Ende des Spritzenkörpers ist ein Anschluss mit Durchgang für ein Druckmedium vorgesehen, wobei es sich bei dem Druckmedium regelmäßig um Druckluft handelt. Die Druckluft dient zum düsenseitigen Ausbringen des Fluidums, wobei der Anschluss an ein offenes Ende des Spritzenkörpers, auf der der Nadel abgewandten Seite des Spritzenkörpers, angeflanscht bzw. anflanschbar ist.

Im Lichte der voranstehenden Ausführungen lässt sich eine herkömmliche Spritze bzw. dessen Spritzenkörper zur Druckbeaufschlagung des im zylindrischen Hohlraum des Spritzenkörpers befindlichen Fluidums nutzen, nämlich dadurch, dass anstelle des Kolbens mit Betätigungsorgan ein Druckluftanschluss vorgesehen ist, über den das Fluidum mit einem einstellbaren gleichmäßigen Druck von einer Druckluftquelle her beaufschlagbar ist. Der Druck ist beliebig einstellbar, sodass eine Anpassung an die konkrete Situation des jeweiligen Eingriffs erfolgen kann.

Die erfindungsgemäße Vorkehrung eines Anschlusses mit Durchgang für ein Druckmedium und dessen Befestigung am Spritzenkörper liefert eine denkbar einfache Ausgestaltung des erfindungsgemäßen Injektionssystems, ohne dass dazu, am Spritzenkörper selbst besondere Maßnahmen bzw. Ausgestaltungen vorzusehen sind.

Für das erfindungsgemäße Injektionssystem ist die Verwendung des Spritzenkörpers herkömmlicher marktüblicher Spritzen von Bedeutung. Diese lassen sich nämlich dann mühelos verwenden, wenn ein besonderer Druckluftanschluss vorgesehen ist, der sich anstelle des Kolbens mit Betätigungsorgan adaptieren lässt. So lässt sich der Anschluss an das der Nadel abgewandte Ende des Spritzenkörpers anflanschen, wozu besondere Befestigungsmittel erforderlich sind.

Der zur Adaption vorgesehene Anschluss umfasst einen Anschlussstutzen durch den hindurch ein Durchgang für die Druckluft ausgebildet ist. Der Anschlussstutzen dient zum unmittelbaren oder mittelbaren Aufschieben, Aufstecken, Aufrasten, Aufschrauben, Einstecken, Einschrauben, etc. eines Druckluftschlauchs. Im Konkreten kann der Anschluss als Luer-Verbindung oder Luer-Lock-Verbindung ausgeführt sein. Die Nutzung eines Luer-Systems hat den Vorteil, dass dieses sich bei zahlreichen Anwendungen hinlänglich bewährt hat und handelsüblich ist.

In vorteilhafter Weise umfasst der Anschluss Befestigungsmittel zum Befestigen am offenen Ende des Spritzenkörpers und Dichtmittel zum Abdichten des im Spritzenkörper ausgebildeten Hohlraums gegenüber der Umgebung. Die Befestigungsmittel dienen zum sicheren Anflanschen. Die Dichtmittel sorgen dafür, dass die aufgegebene Druckluft nicht entweichen kann und wirksam zum Ausbringen des Strömungsmediums, beispielsweise eines Silikonöls, zur Verfügung steht.

Im Konkreten sind die Befestigungsmittel derart ausgebildet, dass sie einen am offenen Ende des Spritzenkörpers ausgebildeten Flansch form- und/oder kraftschlüssig zumindest teilweise umgreifen und/oder übergreifen und/oder untergreifen. Die Dichtmittel umfassen ein zumindest teilweise in den Hohlraum des Spritzenkörpers ragendes Dichtelement und/oder ein zwischen den Befestigungsmitteln und dem Flansch wirkendes Dichtelement. Dabei ist Wesentlich, dass eine wirksame Abdichtung des zylindrischen Hohlraums innerhalb des Spritzenkörpers nach außerhalb realisiert ist.

In weiter vorteilhafter Weise ist der Anschluss im abgedichteten Zustand reversibel oder irreversibel mit dem Spritzenköper verbindbar, insbesondere verspannbar oder verrastbar. So lässt sich bei reversibler Anbringung der Druckluftschlauch einfach vom Spritzenkörper entfernen, wodurch ein sortenreines Entsorgen der unterschiedlichen Materialien begünstigt wird.

Der Anschluss kann ein- oder mehrteilig ausgeführt sein. Bei zweiteiliger oder mehrteiliger Ausgestaltung kann der Anschluss einen Anschlusskörper und einen mit dem Anschlusskörper unter Zwischenschaltung des Spritzenkörpers und/oder des Flanschs in Eingriff bringbaren Arretierungskörper umfassen. Der Anschlusskörper und der Arretierungskörper wirken zusammen und generieren gemeinsam die abdichtende Verbindung unter Zwischenschaltung der Dichtmittel.

Der Arretierungskörper kann durch Schieben oder Schwenken, vorzugsweise von der Seite her, zumindest teilweise um den Anschlusskörper anordenbar sein. Im Rahmen einer besonderen Ausgestaltung lässt sich der Arretierungskörper in den Anschlusskörper einstecken bzw. einschieben und gegebenenfalls einrasten. Die Verrastung kann insbesondere im Rahmen der Ausgestaltung des Injektionssystems zur Einmalverwendung irreversibel ausgeführt sein.

Alternativ ist es denkbar, dass der Arretierungskörper ringartig oder tellerartig ausgebildet und von unterhalb des Anschlusskörpers, vorzugsweise von unterhalb des Flanschs, zumindest teilweise in den Anschlussköper einsteckbar oder durch diesen hindurch steckbar und dabei mit diesem verrastbar ist.

Die zuvor genannten Varianten zum Anflanschen des Anschlusses stellen keine abschließende Auflistung der Verbindungsmöglichkeiten dar. Sie lassen sich beliebig erweitern. Wesentlich ist jedenfalls, dass ein herkömmlicher Spritzenkörper mit dem dort zur Handhabung vorgesehenen Flansch in vorteilhafter Weise genutzt wird, um einen Druckluftanschluss sicher mit dem Spritzenkörper zu verbinden.

In Bezug auf das Material ist es von Vorteil, wenn der Anschluss mit seinem Anschlusskörper und dem Arretierungskörper aus Kunststoff vorzugsweise spritzgusstechnisch hergestellt wird.

Es gibt nun verschiedene Möglichkeiten, die Lehre der vorliegenden Erfindung in vorteilhafter Weise auszugestalten und weiterzubilden. Dazu ist einerseits auf die dem Anspruch 1 nachgeordneten Ansprüche und andererseits auf die nachfolgende Erläuterung bevorzugter Ausführungsbeispiele der Erfindung anhand der Zeichnung zu verweisen. In Verbindung mit der Erläuterung der bevorzugten Ausführungsbeispiele der Erfindung anhand der Zeichnung werden auch im Allgemeinen bevorzugte Ausgestaltungen und Weiterbildungen der Lehre erläutert. In der Zeichnung zeigen
- Fig. 1: in einer schematischen Ansicht eine herkömmliche Spritze mit Spritzenkörper, der zur erfindungsgemäßen Verwendung dienen kann,
- Fig. 2a: in einer schematischen Seitenansicht, geschnitten, ein Ausführungsbeispiel eines erfindungsgemäßen Injektionssystems im Bereich der Verbindung zwischen Spritzenkörper und Anschluss,
- Fig. 2b: in einer schematischen Ansicht, den Gegenstand aus Fig. 2a in einer geschnittenen Draufsicht,
- Fig. 3: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 4a: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 4b: in einer schematischen Ansicht, der Gegenstand aus Fig. 4a in einer geschnittenen Draufsicht,
- Fig. 5: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 6a: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 6b: in einer schematischen Ansicht, der Gegenstand aus Fig. 6a in einer Draufsicht,
- Fig. 7a: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 7b: in einer schematischen Ansicht, der Gegenstand aus Fig. 7a in einer Draufsicht,
- Fig. 8: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 9: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 10a: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 10b: in einer schematischen Ansicht, der Gegenstand aus Fig. 10a in einer Draufsicht,
- Fig. 11a: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 11b: in einer schematischen Ansicht, der Gegenstand aus Fig. 11a in einer Draufsicht,
- Fig. 12a: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 12b: in einer schematischen Ansicht, der Gegenstand aus Fig. 12a in einer teilweise geschnittenen Draufsicht, verriegelt,
- Fig. 12c: in einer schematischen Ansicht, der Gegenstand aus Fig. 12a in einer teilweise geschnittenen Draufsicht, entriegelt,
- Fig. 13: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 14a: in einer schematischen Seitenansicht, geschnitten, ein weiteres Ausführungsbeispiel eines erfindungsgemäßen Injektionssystems im Bereich der Verbindung zwischen Spritzenkörper und Anschluss,
- Fig. 14b: in einer schematischen Ansicht, der Gegenstand aus Fig. 14a in einer Draufsicht,
- Fig. 15a: in einer schematischen Seitenansicht, geschnitten, ein weiteres Ausführungsbeispiel eines erfindungsgemäßen Injektionssystems im Bereich der Verbindung zwischen Spritzenkörper und Anschluss, und
- Fig. 15b: in einer schematischen Ansicht, der Gegenstand aus Fig. 15a in einer Draufsicht,

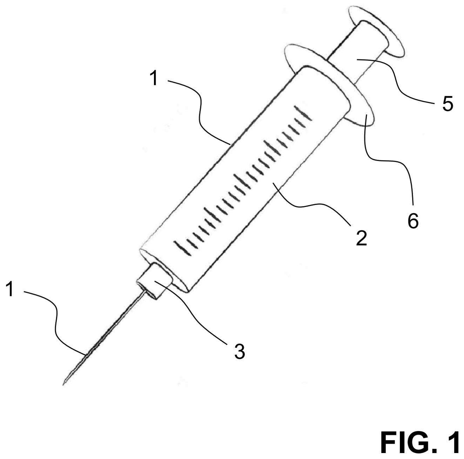

Fig. 1 zeigt in einer schematischen Ansicht eine herkömmliche Spritze mit einem Spritzenkörper 1, der im Inneren einen zylindrischen Hohlraum 2 zur Aufnahme eines Fluidums umfasst. Am vorderen Ende befindet sich eine zylinderförmige Düse 3, an die sich eine Injektionsnadel 4 anschließt.

Am gegenüberliegenden Ende des Spritzenkörpers 1 ragt ein zur Betätigung dienender Kolben 5 aus dem zylindrischen Hohlraum 2 heraus. Am Spritzenkörper 1 ist ein Flansch 6 ausgebildet, der zum Hintergreifen mit den Fingern der Bedienperson, d.h. gemeinsam mit dem Kolben 5 zur Betätigung der Spritze dient.

Für das erfindungsgemäße Injektionssystem wird der Spritzenkörper 1 verwendet.

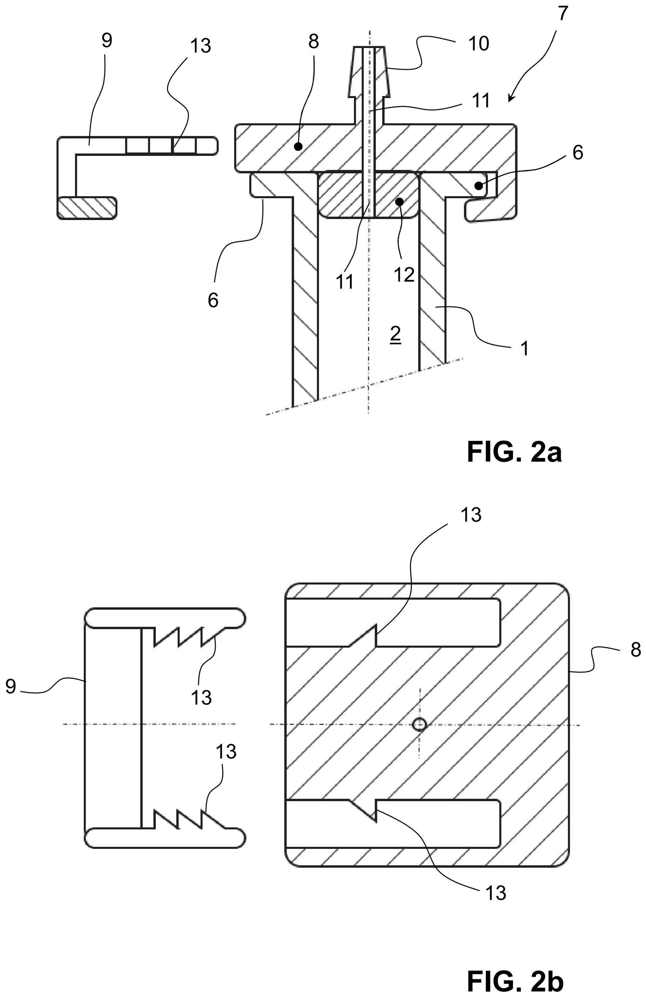

Fig. 2a zeigt in einer geschnittenen Seitenansicht den Bereich des Spritzenkörpers 1 am hinteren Ende. Im Bereich des Flanschs 6 sind Befestigungsmittel 7 angeordnet, wobei die Befestigungsmittel 7 zweiteilig ausgeführt sind.

Ein Anschlusskörper 8 umgreift den Flansch 6 zumindest teilweise.

Von der Seite her wird ein Verriegelungselement 9 in den Anschlusskörper 8 hereingeschoben, so dass der Flansch 6 zumindest von zwei Seiten her umgriffen wird.

Auf der Oberseite des Anschlusskörpers 8 ist ein Anschlussstutzen 10 mit innerem Durchgang 11 ausgebildet, der zum Aufstecken eines in den Figuren nicht gezeigten Druckluftschlauchs dient.

Im Inneren des Spritzenkörpers 1, d.h. im zylindrischen Hohlraum 2, sind Dichtmittel 12 vorgesehen, durch die sich der Durchgang 11 in Form eines Druckluftkanals hindurch erstreckt. Die Dichtmittel 12 dienen zum Abdichten des zylindrischen Hohlraums 2 nach außen.

In den Figuren 2a und 2b ist des Weiteren angedeutet, dass das Verriegelungselement 9 mit dem Anschlusskörper 8 dahingehend zusammenwirkt, dass Eingriffsmittel 13 zum gegenseitigen Verrasten dienen.

Die weiteren Ausführungsbeispiele unterscheiden sich von dem zuvor erörterten Ausführungsbeispiel durch die konkrete Ausgestaltung des Anschlusskörpers 8 nebst Verriegelungselement 9 und durch die Ausgestaltung und Anordnung der Dichtmittel 12. Der Einfachheit halber sind nachfolgend lediglich die konstruktiven Besonderheiten mit Bezugszeichen versehen.

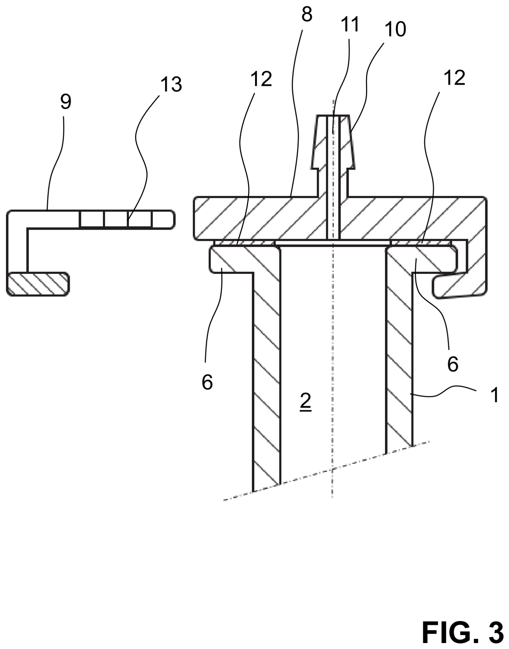

Bei dem Ausführungsbeispiel gemäß Figur 3 sind Dichtmittel 13 vorgesehen, die zwischen dem Anschlusskörper 8 und dem Flansch 6 angeordnet sind und diesen umgreifen. Außerdem hat der Anschlusskörper 8 in Draufsicht eine runde Form.

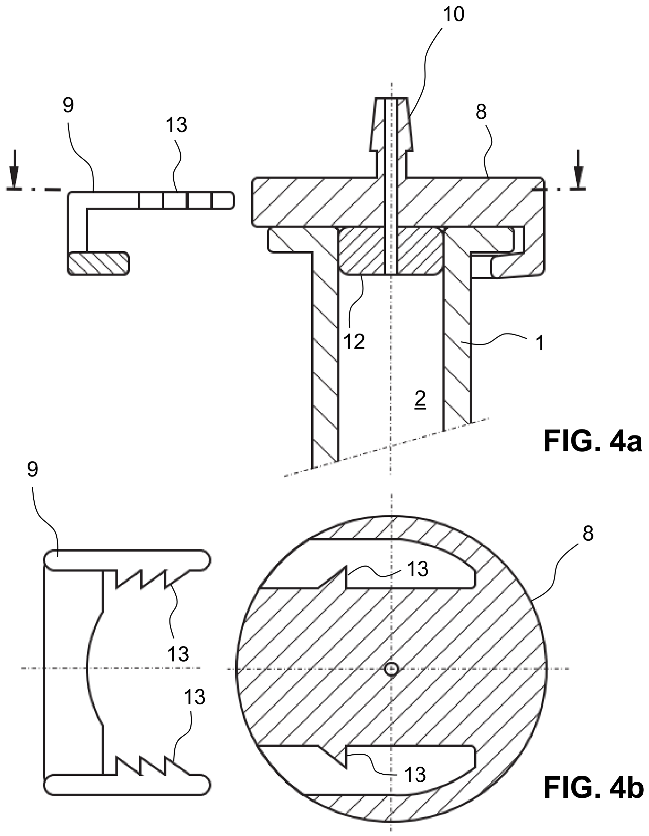

In den Figuren 4a und 4b ist der Anschlusskörper 8 rund ausgeführt, nämlich im Gegensatz zu dem Ausführungsbeispiel gemäß den Figuren 2a und 2b. Die Dichtmittel 12 schließen sich an den Anschlusskörper 8 an und befindet sich im zylindrischen Hohlraum 2.

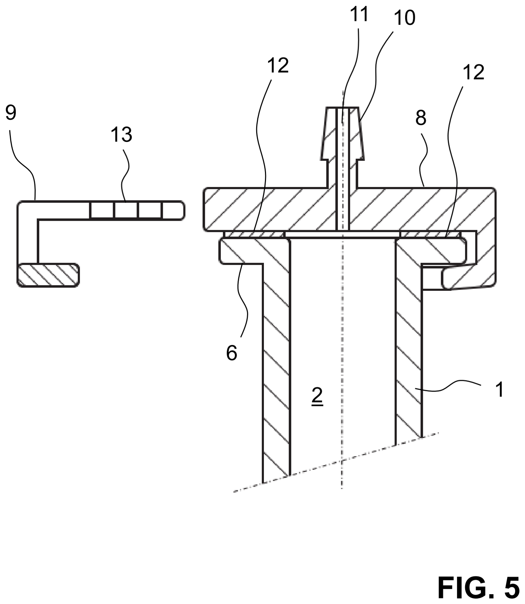

Gemäß der Darstellung in Fig. 5 ist der Anschlusskörper 8 in Draufsicht kreisflächenförmig ausbildet. Die Dichtungen befinden sich zwischen dem Flansch 6 und dem Anschlusskörper 8, wobei dort eine weitere Dichtung im Bereich des Untergriffs des Flansches 6 vorgesehen sein kann.

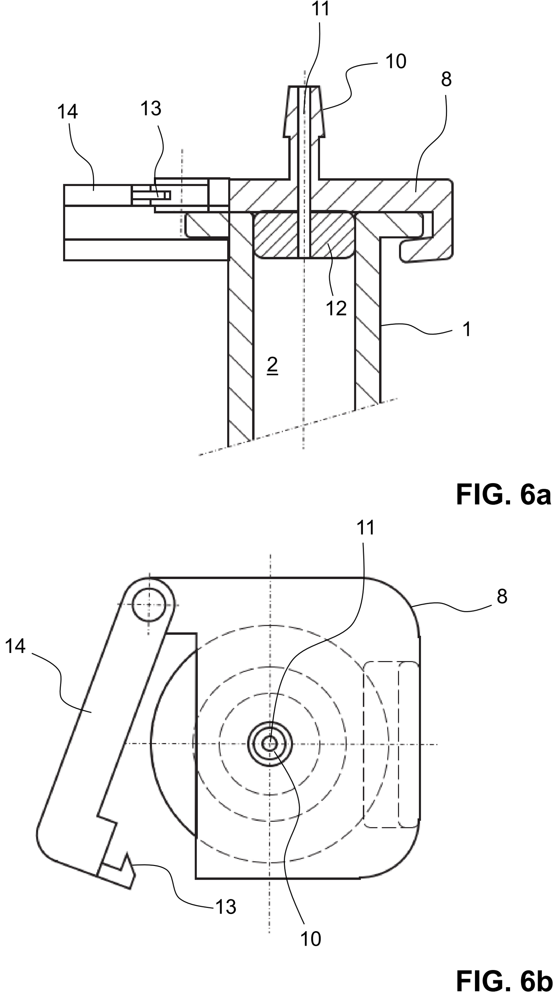

Die Figuren 6a und 6b zeigen eine weitere Ausführungsform eines erfindungsgemäßen Injektionssystems, wobei das Anflanschen des Anschlusskörpers durch ein schwenkbares Verriegelungselement 14 erfolgt. Es ist an seinem freien Ende mit dem Anschlusskörper 8 verrastbar, so dass sich der Anschlusskörper 8 fest mit dem Flansch 6 des Spritzenkörpers 1 verbinden lässt. Dichtmittel 12 befinden sich innerhalb des zylindrischen Hohlraums 2.

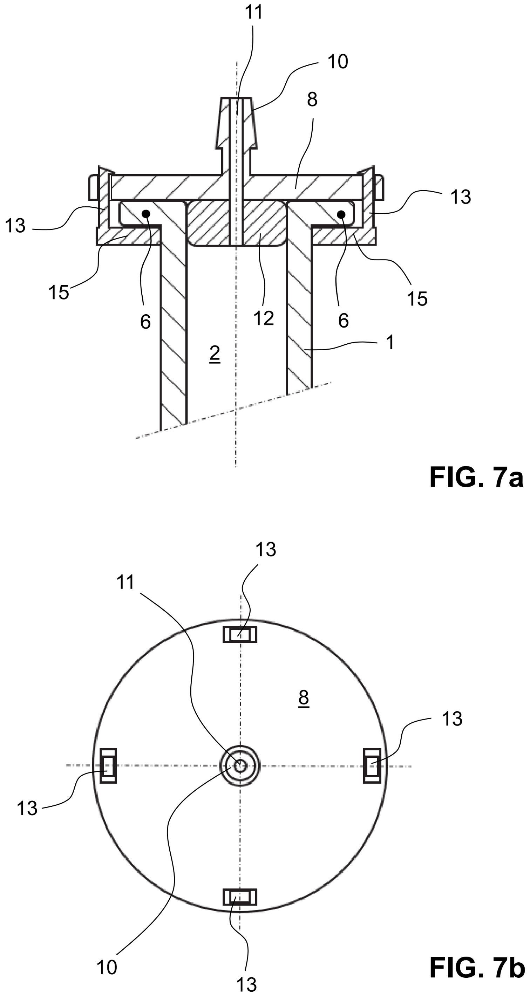

Die Figuren 7a und 7b zeigen ein weiteres Ausführungsbeispiel eines erfindungsmäßen Injektionssystems, wobei der Flansch 6 von unten untergriffen wird, und zwar mit kreisringförmigen Eingriffsmitteln 15, die von unterhalb des Flanschs 6 mit dem Anschlusskörper 8 verrasten. Eingriffsmittel 13 greifen dabei durch Ausnehmungen im Anschlusskörper 8.

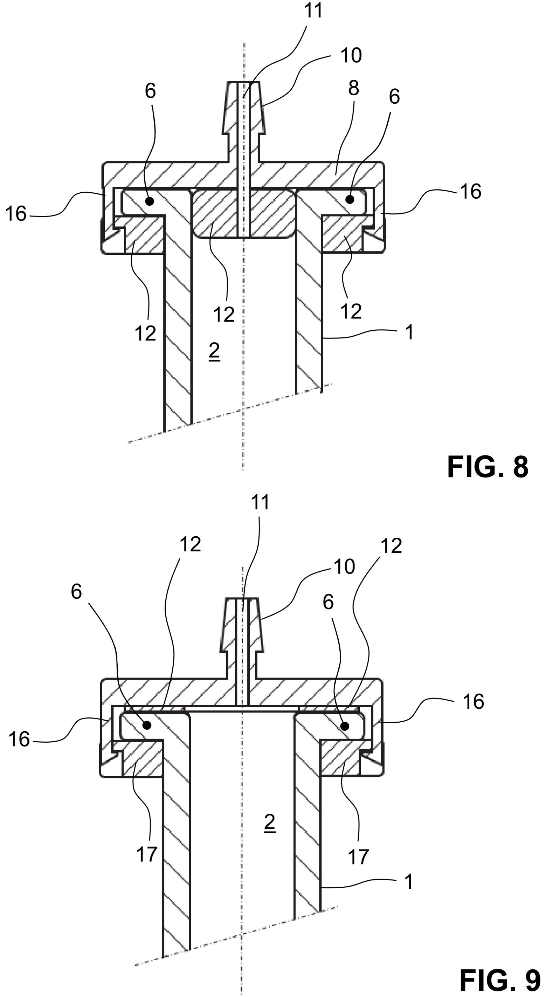

Fig. 8 zeigt ein weiteres Ausführungsbeispiel eines erfindungsgemäßen Injektionssystems, bei dem der Anschlusskörper 8 Rastmittel 16 von oberhalb des Flanschs 6 in ein ringförmiges Dichtungselement 17 greifen. Hier sind sowohl innere Dichtmittel 12 im zylindrischen Hohlraum 2 als auch ein Dichtungselement 17 unterhalb des Flanschs 6 vorgesehen.

Das Ausführungsbeispiel gemäß Fig. 9 unterscheidet sich von dem Ausführungsbeispiel gemäß Fig. 8 dadurch, dass im zylindrischen Hohlraum 2 keine Dichtungsmittel vorgesehen sind. Diese befinden sich vielmehr zwischen dem Flansch 6 und dem Anschlusskörper 8, der äußere, schulterartige Rastmittel 16 umfasst, die sich mit äußeren Dichtmittel, nämlich mit einem ringförmigen Dichtungselement 17, verrasten lassen.

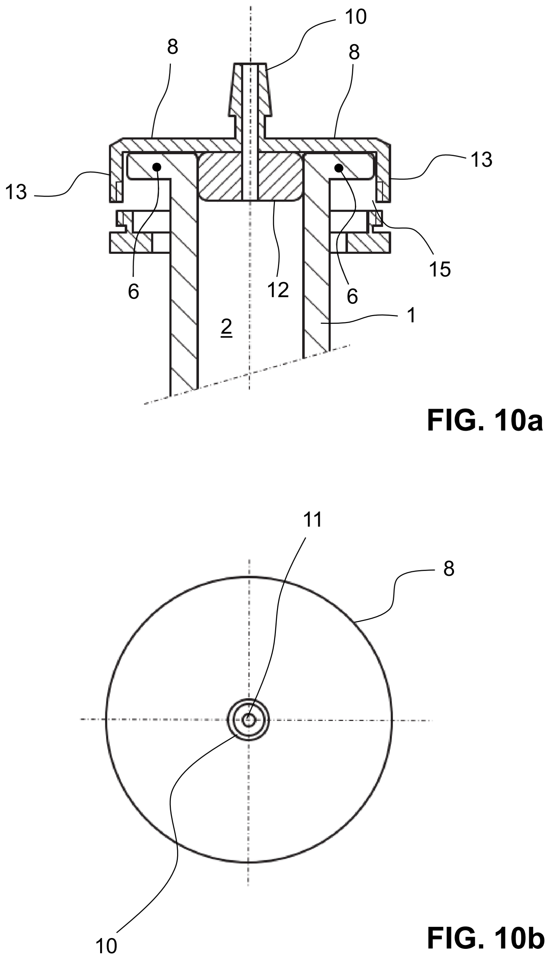

Fig. 10 zeigt ein weiteres Ausführungsbeispiel eines Injektionssystems, bei dem der Anschlusskörper 8 in ein ringförmiges Gegenlager 18 greift, welches von unterhalb des Flanschs 6 auf den Spritzenkörper 1 aufgeschoben ist. Innere Dichtmittel 12 sind vorgesehen. Äußere Dichtmittel in Form eines ringförmigen Dichtungselements 17 können ebenfalls vorgesehen sein.

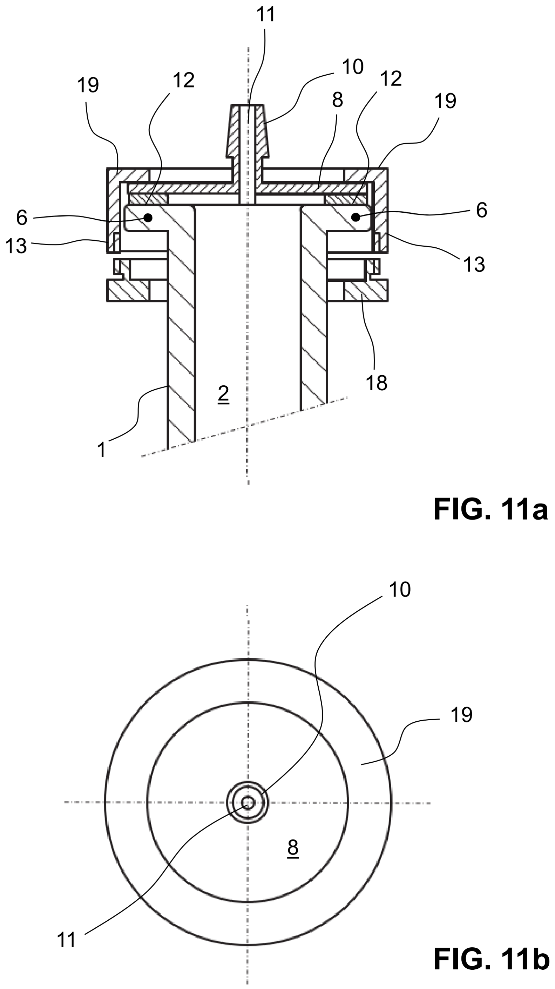

Die Figuren 11a und 11b zeigen ein weiteres Ausführungsbeispiel eines erfindungsgemäßen Injektionssystems, bei dem der Anschlusskörper 8 durch ringförmige Befestigungsmittel 19 umgriffen bzw. übergriffen ist. Die ringförmigen Befestigungsmittel 19 wirken mit einem ringförmigen Gegenlager 18 zusammen. Dichtmittel 12 sind zwischen dem Anschlusskörper 8 und dem Flansch 6 vorgesehen.

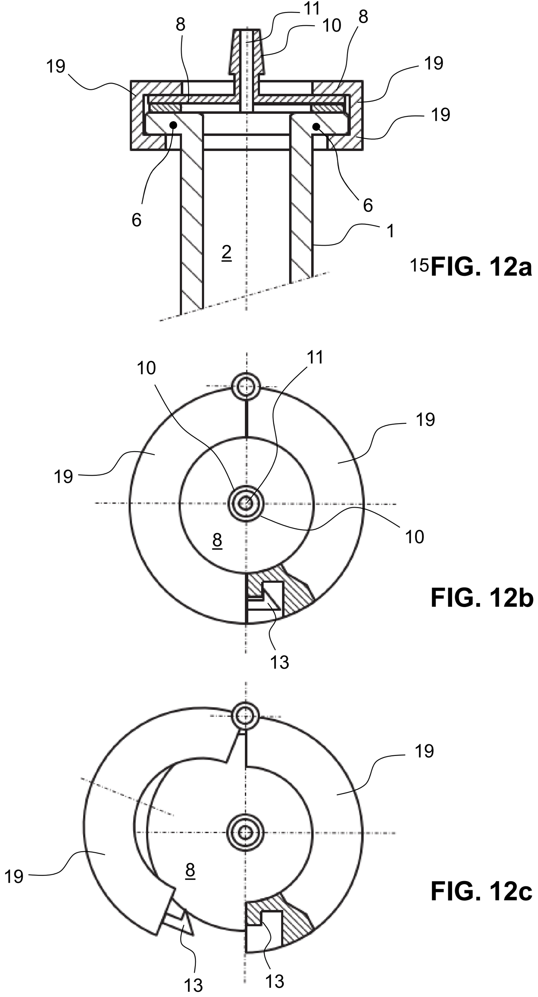

Die Figuren 12a bis 12c zeigen ein Ausführungsbeispiel eines erfindungsgemäßen Injektionssystems, bei dem die ringförmigen Befestigungsmittel 19 zweiteilig und dabei schwenkbar ausgeführt sind. Durch das Zusammenschwenken der beiden Hälften lassen sich diese verrasten und ist eine irreversible Verbindung des Anschlusskörpers 8 mit Anschlussstutzen 10 und dem Flansch 6 hergestellt.

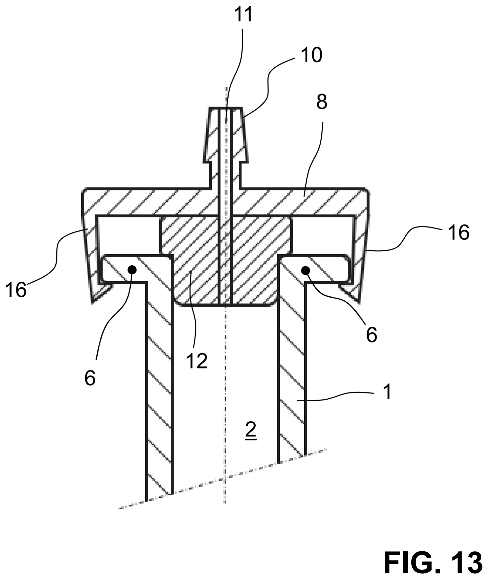

Fig. 13 zeigt ein Ausführungsbeispiel, bei dem der Anschlusskörper 8 über den Flansch 6 streckbar und durch Übergreifen des Flanschs 6 mit Rastmitteln 16 unter Zwischenschaltung der Dichtmittel 12 arretierbar ist. Die Dichtmittel 12 wirken teilweise außerhalb und teilweise innerhalb des zylindrischen Hohlraums 2.

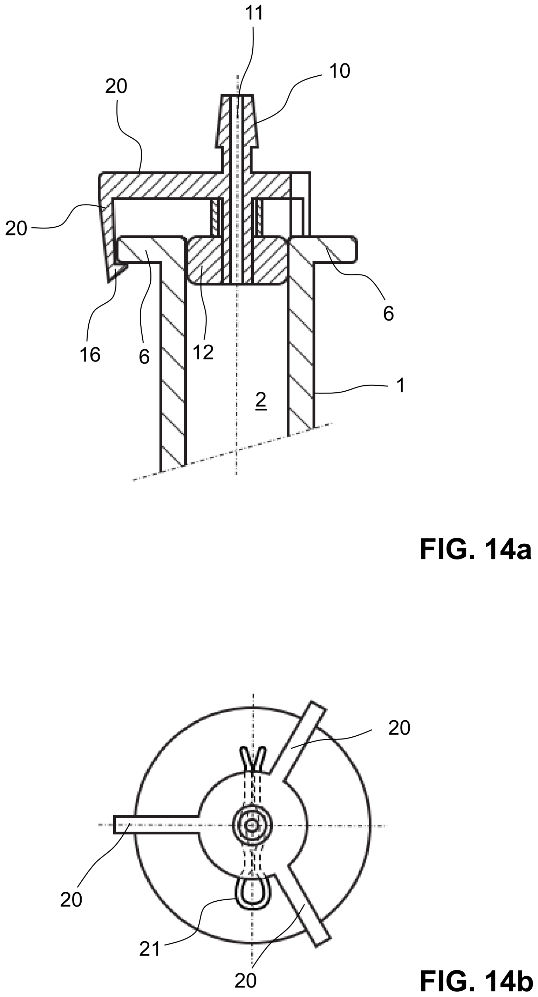

Das Ausführungsbeispiel gemäß den Figuren 14a und 14b zeigt einen Anschlusskörper 8 mit Anschlussstutzen 10, der drei Greifarme 20 mit Rastmitteln 16 umfasst. Der Flansch 6 wird von den Greifarmen 20, an drei Seiten bzw. stellen, äquidistant um- bzw. übergriffen, wobei die Rastmittel 16 den Flansch 6 umgreifen und unter dem Flansch 6 verrasten. Ein Splint 21 kann zu der Positionierung des Anschlusskörpers 8 vorgesehen sein.

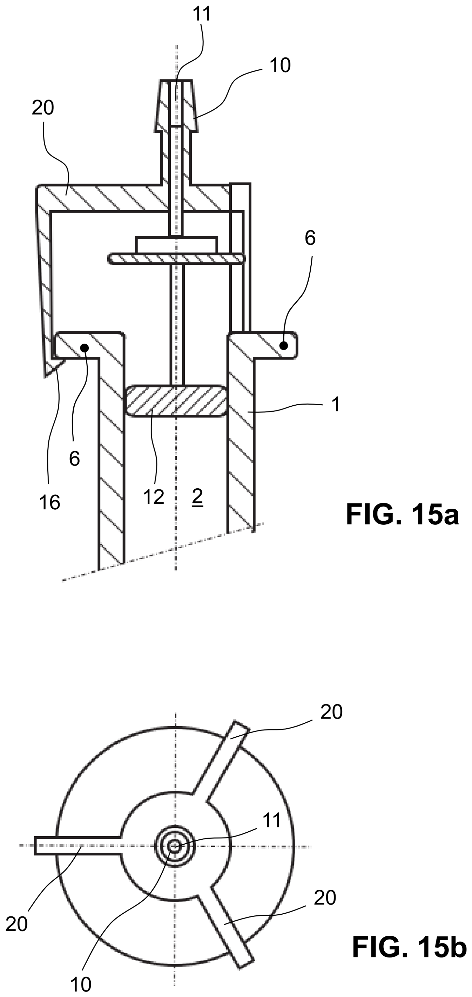

Die Figuren 15a und 15b zeigen ein ähnliches Ausführungsbeispiel wie die Figuren 14a und 14b, nämlich mit einem Anschlusskörper 8, der drei Greifarme 20 umfasst. Über den Anschlussstützen 10 gelangt Druckluft durch eine Art Hülse 22 hindurch in den zylindrischen Hohlraum 2. Innere Dichtmittel 12 sind vorgesehen.

Hinsichtlich weiterer vorteilhafter Ausgestaltungen des erfindungsgemäßen Injektionssystems wird zur Vermeidung von Wiederholungen auf den allgemeinen Teil der Beschreibung sowie auf die beigefügten Ansprüche verwiesen.

Schließlich sei ausdrücklich darauf hingewiesen, dass die voranstehend beschriebenen Ausführungsbeispiele des erfindungsgemäßen Injektionssystems lediglich zur Erörterung der beanspruchten Lehre dienen, diese jedoch nicht auf die Ausführungsbeispiele einschränken.

### Bezugszeichenliste

- 1: Spritzenkörper
- 2: zylindrischer Hohlraum
- 3: zylinderförmige Düse
- 4: Nadel, Injektionsnadel
- 5: Kolben
- 6: Flansch
- 7: Befestigungsmittel
- 8: Anschlusskörper
- 9: Verriegelungselement
- 10: Anschlussstutzen
- 11: Durchgang, Druckluftkammer
- 12: Dichtmittel
- 13: Eingriffsmittel
- 14: schwenkbares Verriegelungselement
- 15: kreisförmige Eingriffsmittel
- 16: Rastmittel
- 17: ringförmiges Dichtelement
- 18: ringförmiges Gegenlager
- 19: ringförmige Befestigungsmittel
- 20: Greifarm
- 21: Splint, Sicherungselement
- 22: Hülse

## Patentansprüche

1. Injektionssystem zur Anwendung bei ophthalmologischen Eingriffen, insbesondere zum Injizieren eines Fluidums in das menschliche oder tierische Auge, vorzugsweise in den hinteren Augenabschnitt, umfassend einen Spritzenkörper (1) mit einem darin ausgebildeten zylindrischen Hohlraum (2) für das Fluidum, einer konus- oder zylinderförmigen Düse (3) zum Anbringen eines Schlauchs oder einer Nadel (4) an den Spritzenkörper (1) und einem am gegenüberliegenden Ende des Spritzenköpers (1) angeordneten Anschluss mit Durchgang (11) für ein Druckmedium, insbesondere Druckluft, zum düsenseitigen Ausbringen des Fluidums, wobei der Anschluss an ein offenes Ende des Spritzenköpers (1) angeflanscht bzw. anflanschbar ist.

2. Injektionssystem nach Anspruch 1, **dadurch gekennzeichnet, dass** der Anschluss einen Teil des Durchgangs (11) bildenden Anschlussstutzen (10) zum unmittelbaren oder mittelbaren Aufschieben, Aufstecken, Aufrasten, Aufschrauben, Einstecken, Einschrauben, etc. eines Druckluftschlauchs aufweist.

3. Injektionssystem nach Anspruch 1 oder 2, **dadurch gekennzeichnet, dass** der Anschluss Befestigungsmittel (7) zum Befestigen am offenen Ende des Spritzenköpers (1) und Dichtmittel (12) zum Abdichten des im Spritzenkörper (1) ausgebildeten Hohlraums gegenüber der Umgebung aufweist.

4. Injektionssystem nach Anspruch 3, **dadurch gekennzeichnet, dass** die Befestigungsmittel (7) einen am offenen Ende des Spritzenkörpers (1) ausgebildeten Flansch form- und/oder kraftschlüssig zumindest teilweise um- und/oder über- und/oder untergreifen und dass die Dichtmittel (12) ein zumindest teilweise in den Hohlraum ragendes Dichtelement und/oder ein zwischen den Befestigungsmitteln (7) und dem Flansch wirkendes Dichtelement umfassen.

5. Injektionssystem nach einem der Ansprüche 1 bis 4, **dadurch gekennzeichnet, dass** der Anschluss im abdichtenden Zustand reversibel oder irreversibel mit dem Spritzenköper (1) verbindbar, insbesondere verspannbar oder verrastbar, ist.

6. Injektionssystem nach Anspruch 4 und ggf. einem der Ansprüche 1 bis 3 und 5, **dadurch gekennzeichnet, dass** der Anschluss zweiteilig ausgeführt ist und einen Anschlusskörper (8) und einen mit dem Anschlusskörper (8) unter Zwischenschaltung des Spritzenkörpers (1) und/oder des Flanschs in Eingriff bringbaren Arretierungskörper umfasst.

7. Injektionssystem nach Anspruch 6, **dadurch gekennzeichnet, dass** der Arretierungskörper durch Schieben oder Schwenken, vorzugsweise von der Seite her, zumindest teilweise um den Anschlusskörper (8) anordenbar und ggf. in den Anschlussköper (8) einsteckbar bzw. einrastbar ist.

8. Injektionssystem nach Anspruch 6, **dadurch gekennzeichnet, dass** der Arretierungskörper ringartig ausgebildet und von unterhalb des Anschlussköpers (8), vorzugsweise von unterhalb des Flansches, zumindest teilweise in den Anschlussköper (8) einsteckbar oder durch diesen hindurch steckbar und dabei mit diesem verrastbar ist.

9. Injektionssystem nach einem der Ansprüche 1 bis 8, **dadurch gekennzeichnet, dass** der Anschluss mit seinem Anschlusskörper (8) und Arretierungskörper aus Kunststoff spritzgusstechnisch hergestellt ist.
